# EUROPEAN PATENT SPECIFICATION

(11) **EP 2 781 203 B1**
(45) Date of publication and mention of the grant of the patent: **23.03.2016**
(21) Application number: 14163140.8
(22) Date of filing: 28.03.2013
(51) Int. Cl.: A61F 2/00, A61M 5/00, D04B 21/12

(54) **Implantable devices including a mesh and an extendable film**
Implantierbare Vorrichtungen mit einem Netz und einer ausdehnbaren Folie
Dispositifs implantables comprenant un treillis et un film perforé

(30) Priority: 30.03.2012 US 201261617820 P; 28.02.2013 US 201313780121
(43) Date of publication of application: 24.09.2014
(62) Divisional of application: 13161521.3
(73) Proprietor: Covidien LP, Mansfield, MA 02048 (US)
(72) Inventor: Stopek, Joshua, Minneapolis, MN 55419-5240 (US); Elachchabi, Amin, Hamden, CT Connecticut 06518 (US); Broom, Daniel, Branford, CT Connecticut 06405 (US)
(74) Representative: Maschio, Antonio

(56) References cited:
- EP-A1- 2 404 571
- EP-A1- 2 543 339
- FR-A1- 2 953 709
- US-A1- 2004 116 774
- US-A1- 2009 105 731
- US-A1- 2009 318 752
- US-A1- 2010 189 764

## Description

This application claims the benefit of and priority to U.S. Provisional Patent Application No. 61/617,820, filed March 30, 2012.

### BACKGROUND

### Technical Field

The present disclosure relates generally to implantable medical devices, and more particularly, to implantable medical devices which include at least one mesh and at least one extendable film positioned on at least a portion of the mesh.

### Background of Related Art

Surgical meshes may be used during both laparoscopic and open surgery for repair of many types of defects and injuries. For example, surgical meshes are commonly used in the repair of hernias. The meshes may be used to provide support to surrounding tissue.

During hernia repair, a mesh may be placed over the entirety of damaged tissue and some of the healthy tissue surrounding the defect. The mesh can be held in place by a fixation device that attaches the mesh to the surrounding tissue. A variety of different fixation devices may be used to anchor the mesh into the tissue. The mesh may further include an additional layer such as a film, for sustained delivery of analgesic agents to the vicinity of the mesh implant for reduction of acute post-operative pain. Integration of films to accommodate unique patient/anatomical features while maintaining the integrity of the film/mesh attachment is desired.

FR 2953709 discloses a prosthesis for treating umbilical hernias with a ring that is fixed at a peripheral outer edge of a flexible circular lattice and maintains the lattice in a flat and unfolded configuration, where lattice is defined by a peripheral outer edge.

### SUMMARY

The invention is disclosed in independent claim 1 and the claims dependent on it. Accordingly, the present disclosure relates to implantable medical devices which include a mesh defining a first plane, and a film including a first portion secured to at least a portion of the mesh and a second portion which extends from the mesh. The mesh may generally be a textile or fabric created to promote tissue ingrowth and/or support injured tissue. The film may generally be polymeric in nature and may be intended to further enhance the ingrowth of tissue into the implant, prevent adhesions of surrounding tissue, deliver therapeutic agents and/or simply provide additional support to the implant. In embodiments, at least a portion of the film extends from the mesh in a fixed position.

The film may include at least one extended portion configured and dimensioned to have a plane different from the plane of the mesh and positioned away from the mesh. The extended portion may be positioned into wound tissue. The extendable portion is stationary. The extended portion is positioned in a second plane different from the first plane of the mesh. The difference between the first plane and the second plane creates a right angle.

The present disclosure also describes implantable medical devices which include a mesh defining a first planar axis, a first film including a first portion secured to at least a portion of the mesh and a plurality of second extended portions extending away from the mesh, and, a second film including a first portion secured to at least a portion of the mesh and a plurality of second extended portions extending away from the mesh.

The first portions of the first and second films may be parallel to each other on the mesh. The first portions of the first and second films criss-cross on the mesh. The films further include movable end portions on at least one of the first and second extended portions.

The implantable medical device may further include at least one therapeutic agent. Methods of delivery of a therapeutic agent are also provided which include implanting a medical device including a mesh defining a first planar axis, a film including a first portion secured to at least a portion of the mesh and a second extended portion having a second planar axis perpendicular to the first planar axis of the mesh, and at least one therapeutic agent; optionally trimming the second extended portion of the film; positioning the second extended portion of the film into a wound site, and closing the wound site.

### BRIEF DESCRIPTION OF THE DRAWINGS

The foregoing objects and advantages of the disclosure will become more apparent from the reading of the following description in connection with the accompanying drawings, in which:
FIG. 1A is a top view of an implantable medical device according to one example described in the present disclosure;
FIG. 1B is a perspective view of the implantable medical device of FIG. 1A with a film extended away from a mesh according to one example described in the present disclosure;
FIG. 2 is a side view of an implantable medical device according to one embodiment described in the present disclosure;
FIGS. 3 is a perspective view of an implantable medical device according to one embodiment described in the present disclosure;
FIG. 4 is a side view of an implantable medical device according to one example described in the present disclosure;
FIG. 5 is a diagram showing the weave of three sheets forming a medical device according to one embodiment described in the present disclosure; and
FIG. 6 is a diagrammatic side view of a device permitting the formation of spiked naps on the medical device of FIG. 5 according to another embodiment described in the present disclosure.

### DETAILED DESCRIPTION

The present disclosure relates to implantable medical devices which include at least one surgical mesh and at least one extendable film. At least a portion of the extendable film being attached to a portion of the mesh. It is envisioned that the extendable portions of the film may be trimmable prior to implantation and/or in situ.

By implantable, the medical devices described herein may be positioned, for any duration of time, at a location within a body, such as within a portion of the abdominal cavity. Furthermore, the terms "implantation" and "implanted" refer to the positioning, for any duration of time, of a medical device at a location within a body, such as within a portion of the abdominal cavity.

The implantable medical devices described herein include at least one surgical mesh. The surgical mesh described herein may include porous fabrics made from intertwined filaments. The filaments may be monofilaments or multi-filaments and, in embodiments, a plurality of multi-filaments may be combined to form yams. The filaments may comprise core/ sheath constructs. The filaments may extend horizontally and vertically in a manner which produces sections where the filaments cross-over one another creating points of common intersection. The surgical mesh may be woven, non-woven, knitted or braided. In some embodiments, the filaments may form two-dimensional or three-dimensional meshes. Some examples of two-dimensional and/or three-dimensional mesh substrates may be found in U.S. Patent No. 7,021,086, U.S. Patent No. 6,596,002, U.S. Patent No. 7,331,199.

Suitable meshes for use in the present disclosure include, for example, a collagen composite mesh such as PARIETEX™ Composite Mesh (commercially available from Tyco Healthcare Group LP d/b/a Covidien). PARIETEX™ Composite Mesh is a 3-dimensional polyester weave with a resorbable collagen film bonded on one side. Another suitable mesh includes Parietex Progrip™ self-fixating mesh (also conmiercially available from Covidien). Parietex Progrip ™ is a polyester mesh which includes poly lactic acid (PLA) grip members. Other suitable meshes include those sold under the names PARIETENE®, PARIETEX™, SURGIPRO™ (all commercially available from Tyco Healthcare Group LP d/b/aCovidien); PROLENE™ (commercially available from Ethicon, Inc.); MARLEX@, DLTLEX@, 3D MAX@ mesh, PERFIX@ plug, VENTRALEX®, and KUGEL@ patch (all commercially available from C.R. Bard, Inc.); PROLITE™, PROLITE ULTRA™ (all commercially available from Atrium Medical); COMPOSIX@, SEPRAMESH®, and VISILEX@ (all commercially available from Davol, Inc.); and DLTALMESH@, MYCROMESH®, and INFINIT® mesh (all commercially available from W.L. Gore). Additionally, meshes within the scope and context of this disclosure may include biologic materials such as allografts (i.e., AlloDerm® Regenerative Tissue Matrix from Lifecell), autografts, and xenografts (i.e., PERMACOL™, from Covidien). In alternate embodiments, processed/purified tissues may also be employed.

In general, medical devices of the present disclosure comprise synthetic, or natural materials, absorbable or non-absorbable materials and suitable combinations thereof.

In certain embodiments, Parietex™ Composite Mesh or Parietex™ Pro-grip may be utilized in accordance with the present invention.

In certain embodiments, the medical device may be a surgical mesh knitted on a warp knitting machine, of the tricot or Raschel type, with at least three sheets or warps of yarn and as many guide bars.

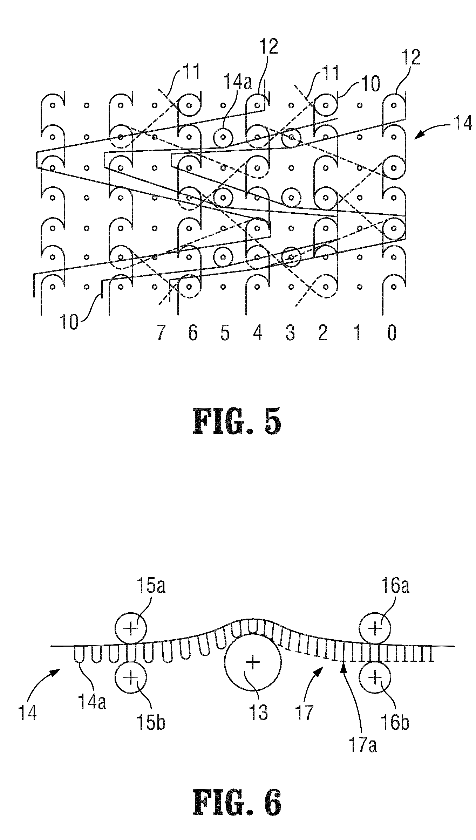

A rear bar is threaded, one guide full and one guide empty, with first mono- or multi-filaments 10 of a biocompatible polymer as represented as a solid line in FIG. 5. An intermediate bar is threaded, one guide full, three guides empty, with second mono- or multi-filaments 11 of a biocompatible polymer as represented as a broken line in FIG. 5. The intermediate bar works in such a way as to obtain a zigzag openwork pattern between the columns of meshes. Finally, a front bar is threaded, one guide full, one guide empty, and works in a chain stitch with third mono- or multi-filaments 12 a biocompatible polymer as represented by a thin line in FIG. 5. The third filament 12, i.e., a chain stitch, imprisons first filament 10 and maintains the length of the mesh while contributing to the formation of the mesh with the intermediate sheet formed by the second filament 11. The different filaments may form yarns and may be worked according to the following chart:

| **Warp →** | **Rear bar I** | **Intermediate bar II** | **Front bar III** |
|---|---|---|---|
| **Raschel** → | **Front bar I** | **Intermediate bar II** | **Rear bar III** |
| | 7 | 3 | 1 |
| | 7 | 2 | 0 |
| | _ | _ | _ |
| | 3 | 4 | 0 |
| | 4 | 5 | 1 |
| | _ | _ | _ |
| | 0 | 1 | |
| | 0 | 0 | |
| | _ | _ | |
| | 4 | 2 | |
| | 3 | 3 | |
| | | _ | |
| | | 1 | |
| | | 0 | |
| | | _ | |
| | | 4 | |
| | | 5 | |

The rear bar places the first filament or yarn in partial weft under the chain stitch and "thrown" onto the needle not forming a chain stitch. For this reason, at the next row, the needle not forming a chain stitch not being supplied permits escape of the filament which forms a loop 14*a* projecting from the front face of the mesh.

The threading-one guide full, three guides empty-in the intermediate bar, associated with the displacement, makes it possible to form a light ground texture, stable in width, and open-worked to permit good tissue integration.

The mesh 14 thus obtained may be provided with loops 14*a* (FIG. 6) which may be perpendicular to one of the mesh surfaces. Loops 14a may also include a rigidity and hold at a right angle which may be obtained by the rigidity or nerve of the filaments employed. This rigidity may be necessary for the subsequent formation of grip members which ensure a grip function to at least a portion of the implantable medical device.

On leaving the loom, mesh 14 may be subjected to a thermosetting operation which stabilizes the mesh length and width. The mesh may then be subjected to a phase of formation of the grip members consisting, as is shown in FIG. 6, in passing the mesh over a cylinder 13 containing an electrical heating resistor. Mesh 14 is pressed flat on cylinder 13 by two pairs of rollers, upstream 15*a*, 15*b* and downstream 16*a*, 16*b*, respectively, which are vertically displaceable for controlling this pressing force.

This control as well as that of the temperature of the resistor placed in cylinder 13 and of the speed of movement of mesh 14 across cylinder 13 make it possible to melt the head of each of loops 14*a* so that each loop 14*a* forms two grip members 17.

Each grip member 17 thus may have a substantially rectilinear body protruding perpendicularly with respect to mesh 14 and, at the free end of this body, a head 17*a* of greater width than that of the body. Head 17*a* has a generally spheroidal shape or a mushroom shape. Grip member 17 gives mesh 14 the ability to attach to tissue when implanted. In addition, grip members 17 may attach to other portions of mesh 14 when folded or rolled. The grip members may be positioned along any portion of the mesh and in any quantity and/or configuration. For example, in some embodiments, the grip members may be positioned on the same portion of the mesh as the film. In other embodiments, the grip members may be positioned on a different portion of the mesh which does not include the film.

The medical devices described herein further include a film layer which may be made from any biocompatible material.

Some non-limiting examples of bioabsorbable materials used to form the film include polymers selected from the group consisting of aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

More specifically, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable bioabsorbable materials may include but are not limited to poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; mucilage, pectin; and combinations thereof.

The term "collagen" is meant to include any type of collagen, whether natural or synthetic, of human or animal origin, such as, for example, enriched human collagen of type I, human collagen of type III, also enriched, human collagen of type 1+111 or of type IV or other collagens such as animal collagen of type I or of type I+III. The collagen may be oxidized or non-oxidized. In certain embodiments, the collagen may be oxidized without crosslinking. For example, native collagen may be dipped in an acid solution and/or washed, to eliminate the telopeptides, notably by pepsin digestion.

The collagen may also be modified by oxidative cleavage. For this purpose periodic acid or one of its salts can be used, applying the technique described by M. TARDY et al. (FR-A-2 601 371 and U.S. Pat. No. 4,931,546).

It is recalled briefly that this technique consists of mixing the collagen in acid solution with a solution of periodic acid or one of its salts at a concentration of between about 1 and about 10⁻⁵ M, in embodiments between about 5x10⁻³ and about 10-1 M, at a temperature of between about 10°C and about 25°C for about 10 minutes to about 72 hours.

This process breaks down some of the collagen's components, these being hydroxylysine and the sugars, thus creating reactive sites without causing crosslinking.

The oxidative cleavage of collagen allows moderate cross-linking later in the collagenic material but does not exclude the possibility of providing this function by other means of moderate cross-linking, for example by beta or gamma irradiation, or other agents of moderate cross-linking, for example chemical reagents at suitably low and non-toxic doses.

For some applications, the polymer film layers described herein may include collagen which is not oxidized or a mixture of non-oxidized and oxidized collagens. In other embodiments, the film layer may include about 25-75% by weight oxidized collagen and about 25-75% by weight non-oxidized collagen.

Both the mesh and/or the film may further consist of at least one optional ingredient. Some examples of suitable optional ingredients include emulsifiers, viscosity enhancers, dyes, pigments, fragrances, pH modifiers, wetting agents, plasticizers, antioxidants, and the like. The optional ingredients may represent up to about 10% of the mesh and/or film by weight.

In some embodiments, the film may include at least one plasticizer, i.e., glycerol, PEG, etc. For instance, the film may include collagen, and at least one of PEG and glycerol.

The film and/or surgical mesh may be pre-formed or cut into any suitable shape, such as for example, round, square, star shaped, octagonal, rectangular, polygonal, triangle, u-shaped, and oval. In embodiments, the film may be pre-formed or cut into rectangular strips.

The films described herein may be formed by any suitable method known to those skilled in the art. In certain embodiments, a solution may be formed which includes the suitable polymeric material and any optional ingredients. The solution may be cast bulk sheet stock, spray coated using an ultrasonic sprayer, extruded, molded and the like, to form the films described herein. Suitable solvents for making polymer solutions include, without limitation, methylene chloride, chloroform, N-methylpyrrolidone, tetrahydrofuran, dimethylformamide, methanol, ethanol, hexanes, acetone, water and combinations thereof.

In some embodiments, the film may be cast directly on a portion of the mesh surface. In other embodiments, the film may be spray coated directly on a portion of the mesh. In still other embodiments, the film may be formed before being connected to the mesh.

In certain embodiments, the film may be created using a spraying technique, such as ultrasonic spraying. For example, the medical device as described herein may be fabricated by passing one or more solutions containing the polymer(s) materials suitable for forming the film and optionally one or more therapeutic agents through an ultrasonic spray nozzle to form droplets. The droplets may be mixed while falling towards or being deposited onto an inert substrate, such as silicone sheet, or a portion of the mesh to form the film. In some embodiments, prior to spraying the film, an inert substrate may be positioned on the portion of the mesh, preventing film attachment. Thus, upon formation of the film, the film may adhere to the portions of the mesh which are not covered by the inert substrate and the film will not fixedly attach to the portions of the mesh which are covered by the inert substrate. The inert substrate may be removed from the implant prior to implantation.

The films disclosed herein may include a single layer or multiple layers. In other embodiments, at least one of the layers includes at least one therapeutic agent.

The implantable medical devices of the present disclosure may include at least one therapeutic agent. The therapeutic agent may be included in any portion of the implant including the mesh, the film and/or the perforation. The term "therapeutic agent", as used herein, is used in its broadest sense and includes any substance or mixture of substances that provides a beneficial, therapeutic, pharmacological, and/or prophylactic effect. The agent may be a drug which provides a pharmacological effect.

The term "drug" is meant to include any agent capable of rendering a therapeutic affect, such as, anti-adhesives, antimicrobials, analgesics, antipyretics, anesthetics (e.g. local and systemic), antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes. It is also intended that combinations of agents may be used.

Other therapeutic agents, which may be included as a drug include: anti-fertility agents; parasympathomimetic agents; psychotherapeutic agents; tranquilizers; decongestants; sedative hypnotics; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; and immunological agents.

Other examples of suitable agents, which may be included in the devices described herein include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, and RNAi; oligonucleotides; polynucleotides; and ribozymes.

Some specific non-limiting examples of water-soluble drugs that may be used in the present implantable devices include, lidocaine, bupivicaine, tetracaine, procaine, dibucaine, sirolimus, taxol, chlorhexidine, polyhexamethylene, thiamylal sodium, thiopental sodium, ketamine, flurazepam, amobarbital sodium, phenobarbital, bromovalerylurea, chloral hydrate, phenytoin, ethotoin, trimethadione, primidone, ethosuximide, carbamazepine, valproate, acetaminophen, phenacetin, aspirin, sodium salicylate, aminopyrine, antipyrine, sulpyrine, mepirizole, tiaramide, perixazole, diclofenac, anfenac, buprenorphine, butorphanol, eptazocine, dimenhydrinate, difenidol, dl-isoprenaline, chlorpromazine, levomepromazine, thioridazine, fluphenazine, thiothixene, flupenthixol, floropipamide, moperone, carpipramine, clocapramine, imipramine, desipramine, maprotiline, chlordiazepoxide, clorazepate, meprobamate, hydroxyzine, saflazine, ethyl aminobenzoate, chlorphenesin carbamate, methocarbamol, acetylcholine, neostigmine, atropine, scopolamine, papaverine, biperiden, trihexyphenidyl, amantadine, piroheptine, profenamine, levodopa, mazaticol, diphenhydramine, carbinoxamine, chlorpheniramine, clemastine, aminophylline, choline, theophylline, caffeine, sodium benzoate, isoproterenol, dopamine, dobutamine, propranolol, alprenolol, bupranolol, timolol, metoprolol, procainamide, quinidine, ajmaline, verapamil, aprindine, hydrochlorothiazide, acetazolamide, isosorbide, ethacrynic acid, captopril, enalapril, delapril, alacepril, hydralazine, hexamethonium, clonidine, bunitrolol, guanethidine, bethanidine, phenylephrine, methoxamine, diltiazem, nicorandil, nicametate, nicotinic-alcohol tartrate, tolazoline, nicardipine, ifenprodil, piperidinocarbamate, cinepazide, thiapride, dimorpholamine, levallorphan, naloxone, hydrocortisone, dexamethasone, prednisolone, norethisterone, clomiphene, tetracycline, methyl salicylate, isothipendyl, crotamiton, salicylic acid, nystatin, econazole, cloconazole, vitamin B₁, cycothiamine, vitamin B₂, vitamin B₃, vitamin B₅, vitamin B₆, vitamin B₇, vitamin B₉, vitamin B₁₂, vitamin C, nicotinic acid, folic acid, nicotinamide, calcium pantothenate, pantothenol, panthetin, biotin, ascorbic acid, tranexamic acid, ethamsylate, protamine, colchicine, allopurinol, tolazamide, glymidine, glybuzole, metoformin, buformin, orotic acid, azathioprine, lactulose, nitrogen mustard, cyclophophamide, thio-TEPA, nimustine, thioinosine, fluorouracil, tegafur, vinblastine, vincristine, vindesine, mitomycin C, daunorubicin, aclarubicin, procarbazine, cisplatin, methotrexate, benzylpenicillin, amoxicillin, penicillin, oxycillin, methicillin, carbenicillin, ampicillin, cefalexin, cefazolin, erythromycin, kitasamycin, chloramphenicol, thiamphenicol, minocycline, lincomycin, clindamycin, streptomycin, kanamycin, fradiomycin, gentamycin, spectinomycin, neomycin, vanomycin, tetracycline, ciprofloxacin, sulfanilic acid, cycloserine, sulfisomidine, isoniazid, ethambutol, acyclovir, gancyclovir, vidabarine, azidothymidine, dideoxyinosine, dideoxycytosine, morphine, codeine, oxycodone, hydrocodone, cocaine, pethidine, fentanyl, polymeric forms of any of the above drugs and any combinations thereof.

In some embodiments, the therapeutic agent may include an anesthetic, i.e., bupivicaine, lidocaine, benzocaine, and the like.

Although the above therapeutic agents have been provided for the purposes of illustration, it should be understood that the present disclosure is not so limited. In particular, although certain therapeutic agents are specifically referred to above, the present disclosure should be understood to include analogues, pro-drugs, derivatives and conjugates of such agents.

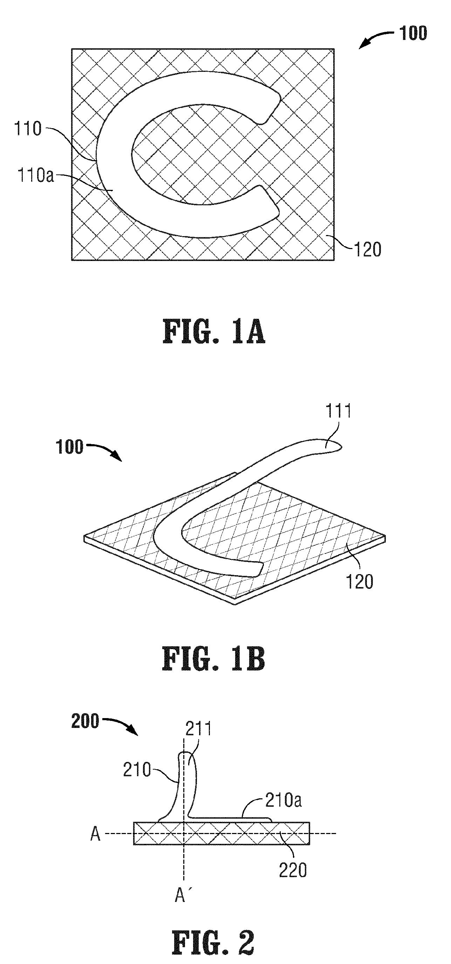

Turning now to FIG. 1A, implantable medical device 100 is illustrated including film 110 positioned on mesh 120 wherein film 110 covers a portion of at least one side of mesh 120. Film 110 includes first portion 110a which is secured to mesh 120 and extended portion 111 which may initially be positioned in the same plane as mesh 120 (FIG. 1A). In some examples, extended portion 111 of film 110 may be movable to extend away form mesh 120 (Fig. 1B). In some embodiments, extended portion 111 of film 110 may extend away from mesh 120 in a fixed position (Fig. 2).

First portion 110a of film 110 may be permanently attached to mesh 120 by any suitable means in the art and as described herein. Although film 110 is shown initially having a generally "u" shape, it is envisioned that film 110 may be formed into any suitable shape and as mentioned herein.

Referring to FIG. 2, implantable medical device 200 is shown including film 210 including finger(s) 211 which is configured and dimensioned to extend upward into a vertical or substantially perpendicular configuration relative to planar axis A of mesh 220. First portion 210a of film 210 is shown fixedly attached to mesh 220. Finger 211 extends away from mesh 220 in a fixed position and includes second planar axis A' which is different from planar axis A of mesh 220. Finger 211 may be positioned along a wound area or incision space to allow for direct treatment of tissue. For example, during a hernia wound repair, the mesh may be placed directly at or near the wound site so the finger(s) may be positioned perpendicularly to planar axis A of mesh 220 and directly on either side of the wound to provide therapeutic agents to the wound (not shown in Fig. 2). In alternative embodiments, the finger may be used to wrap around sensitive tissues and/ or organs.

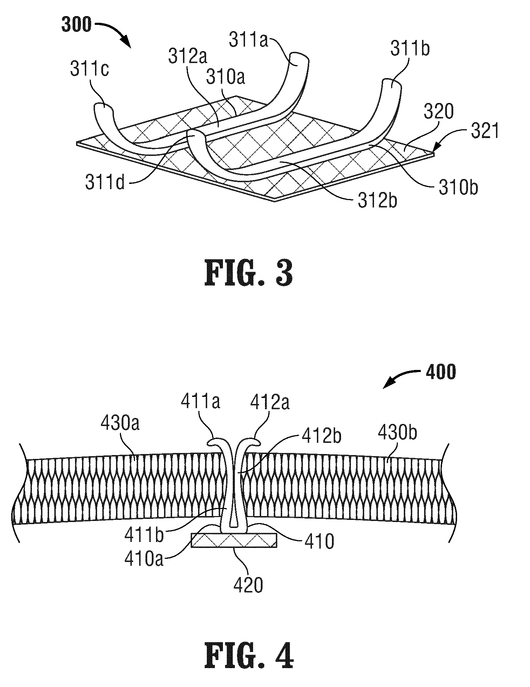

Turning now to FIG. 3, implantable medical device 300 is shown including first film 310a and second film 31 Ob positioned on mesh 320 wherein first and second films 310a, 310b are fixedly attached to mesh 320 via first inner portion 312a of film 310a and second inner portion 312b of second film 310b. Each film 310a and 310b also includes a plurality of fingers 31 la-d which extend away from mesh 320. First fingers 311a, 311c are in a fixed position relative to mesh 320 on first film 310a. Second fingers 311b, 311d are in a fixed position relative to mesh 320 on second film 310b.

Although first and second films 310a and 310b are shown parallel to one another on film 320, it is envisioned that first and second films 310a, 310b can be positioned on mesh 320 in other configurations. For instance, the first film and the second film may criss-cross. In another example, the first and second film may be positioned along the outer perimeter of the mesh.

As shown, films 310a and 310b may extend beyond the outer edge 321 of mesh 320. This configuration may be useful in hernia repair wherein at least one of films 310a and 310b represent an anti-adhesion barrier film. By extending past the outer perimeter of mesh 320, anti-adhesive film 310a and/or 310b may not only prevent adhesion along the top side of mesh 320, but also along the side edges of mesh 320 along the perimeter. Although film 310 is shown having a rectangular shape, it is envisioned that film 310 may be formed into other shapes as mentioned above.

FIG. 4 illustrates film 410 attached to mesh 420 and positioned between approximated first and second wound tissue 430a and 430b, respectively. As depicted in FIG. 4, first portion 410a of film 410 is fixedly attached to mesh 420, both of which are positioned on a first side of tissue 430a and 430b. In addition first extended portion 411b of film 410 and second extended portion 412b of film 410 extend away from mesh 420 through approximated wound tissues 430a and 430b and onto a second side of wound tissue 430a and 430b which is opposite the first side of the tissue. It is envisioned that by extending through the tissue to a second, opposite side of the wound, the extended portions of the film may be used to enhance the strength of the wound closure and/or deliver a therapeutic agent such as an anesthetic and/or analgesic to reduce the pain and/or inflammation frequently associated near the exterior of a closed wound site.

First extended portion 411b includes end portions 411a and second extended portion 412b includes second end portions 412a. First and second end portions 411a, 412a are positioned on a second side of the wound and may be used to anchor the implant to the wound tissue. It is envisioned that any combination of first and second extended portion and first and second end portions may be movable and/or in a fixed position relative to the mesh. For instance, in some embodiments, the implants described herein may include first and second extended portions which may be fixed generally perpendicular to the planar axis of the mesh, and first and second end portions which may be free to move as needed at or near the wound site. In an alternative embodiment, the first extended portion may be in a fixed position and the second extended portion may be movable, and the both end portions are movable. Any combination of movable and fixed portions is envisioned.

The implants described herein may be useful in many endoscopic, laparoscopic, arthroscopic, endoluminal, transluminal, and/or open surgical procedures. Some examples include hernia repair, repair of vaginal prolapse, ligament repair, tendon repair, and the like. Although the polymeric films described herein may be made from any biocompatible materials, in certain procedures, the film layers may be made from anti-adhesive materials.

Methods of delivery of a therapeutic agent are also provided herein which includes implanting a medical device including a mesh, and a film positioned on at least a portion of the mesh, wherein at least a portion of the film extends away from the mesh in a fixed position; optionally trimming a portion of the extended film to a predetermined configuration; and positioning the extended film and mesh onto a wound site. The methods described herein may further include folding the medical device; inserting the folded medical device through an instrument having an elongated tubular member; and positioning the medical device at or near the wound site.

It will be understood that various modifications may be made to the embodiments disclosed herein. For example, the medical device may be folded prior to being delivered into the body via a cannula, trocar or laparoscopic delivery device. In another example, the medical devices described herein may be sterilized and packaged into using any suitable sterilization process, i.e., gamma radiation, and any suitable medical device package, i.e., an injectable medical device package. In still other examples, the implants described herein may include more than one film, mesh, finger, extended portion, and/or therapeutic agent. Thus, those skilled in the art will envision other modifications within the scope of the claims.

## Claims

1. An implantable medical device (200; 300) comprising:
a mesh (220) defining a first plane, and
a film (210) including a first end portion (210a) secured to at least a portion of a surface of the mesh (220) and a second extended end portion (211);
**characterised in that**:
the second extended end portion (211) of the film (210) is stationary;
the second extended end portion (211) of the film (210) defines a second plane different from the first plane of the mesh (220); and
the first plane defined by the mesh (220) and the second plane defined by the film (210) creates a right angle.

2. The implantable medical device (200) according to claim 1, wherein the second extended end portion (211) of the film (210) is disposed within the bounds of an outer edge of the mesh (220).

3. The implantable medical device (200) according to any preceding claim, wherein the implant (200) further comprises at least one therapeutic agent.

4. The implantable medical device (200) according to any preceding claim, wherein the second extended end portion (211) of the film (210) is trimmable.

5. The implantable medical device (200) according to any preceding claim, wherein the film extends beyond the outer edge of the mesh (220).

6. The implantable medical device (200) according to any preceding claim, wherein the mesh (220) further comprises at least one grip-member (17).

## Patentansprüche

1. Implantierbare medizinische Vorrichtung (200; 300), umfassend:
ein Netz (220), das eine erste Ebene definiert, und
eine Folie (210), die einen ersten Abschnitt (210a), der an mindestens einem Abschnitt einer Oberfläche des Netzes (220) befestigt ist, und einen zweiten ausgedehnten Endabschnitt (211) aufweist;
**dadurch gekennzeichnet, dass**:
der zweite ausgedehnte Endabschnitt (211) der Folie (210) unbeweglich ist;
der zweite ausgedehnte Endabschnitt (211) der Folie (210) eine zweite Ebene definiert, die sich von der ersten Ebene des Netzes (220) unterscheidet; und
die erste durch das Netz (220) definierte Ebene und die zweite durch die Folie (210) definierte Ebene einen rechten Winkel erzeugen.

2. Implantierbare medizinische Vorrichtung (200) nach Anspruch 1, wobei der zweite ausgedehnte Endabschnitt (211) der Folie (210) innerhalb der Begrenzung einer Außenkante des Netzes (220) angeordnet ist.

3. Implantierbare medizinische Vorrichtung (200) nach einem der vorhergehenden Ansprüche, wobei das Implantat (200) ferner mindestens einen therapeutischen Wirkstoff umfasst.

4. Implantierbare medizinische Vorrichtung (200) nach einem der vorhergehenden Ansprüche, wobei der zweite ausgedehnte Endabschnitt (211) der Folie (210) trimmbar ist.

5. Implantierbare medizinische Vorrichtung (200) nach einem der vorhergehenden Ansprüche, wobei die Folie sich über die Außenkante des Netzes (220) hinaus erstreckt.

6. Implantierbare medizinische Vorrichtung (200) nach einem der vorhergehenden Ansprüche, wobei das Netz (220) ferner mindestens ein Griffelement (17) aufweist.

## Revendications

1. Dispositif médical implantable (200 ; 300)
comprenant :
un treillis (220) définissant un premier plan,
un film (210) comprenant une première partie d'extrémité (210a) fixée à au moins une partie d'une surface du treillis (220) et une seconde partie d'extrémité étendue (211) ;
**caractérisé en ce que** :
la seconde partie d'extrémité étendue (211) du film (210) est fixe ;
la seconde partie d'extrémité étendue (211) définit un second plan différent du premier plan du treillis (220) ; et
le premier plan défini par le treillis (220) et le second plan défini par le film (210) créent un angle droit.

2. Dispositif médical implantable (200) selon la revendication 1, dans lequel la seconde partie d'extrémité étendue (211) du film (210) est disposée dans les limites d'un bord externe du treillis (220).

3. Dispositif médical implantable (200) selon l'une quelconque des revendications précédentes, dans lequel l'implant (200) comprend en outre au moins un agent thérapeutique.

4. Dispositif médical implantable (200) selon l'une quelconque des revendications précédentes, dans lequel la seconde partie d'extrémité étendue (211) du film (210) peut être rognée.

5. Dispositif médical implantable (200) selon l'une quelconque des revendications précédentes, dans lequel le film s'étend au-delà du bord externe du treillis (220).

6. Dispositif médical implantable (200) selon l'une quelconque des revendications précédentes, dans lequel le treillis (220) comprend en outre au moins un élément de préhension (17).
